(19) Europäisches Patentamt
European Patent Office
Office européen des brevets

(11) **EP 2 977 074 B1**

(12) **EUROPEAN PATENT SPECIFICATION**

(45) Date of publication and mention
of the grant of the patent:
**01.05.2019 Bulletin 2019/18**

(51) Int Cl.:
*A61M 27/00* (2006.01)  *A61F 2/04* (2013.01)

(21) Application number: **14178201.1**

(22) Date of filing: **23.07.2014**

(54) **A ureteral stent**

Harnröhrenstent

Endroprothèse urétérale

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**27.01.2016 Bulletin 2016/04**

(60) Divisional application:
**19157690.9**

(73) Proprietor: **Coloplast A/S
3050 Humlebaek (DK)**

(72) Inventors:
• **Seguy, Sebastien**
  **46300 Gourdon (FR)**
• **Beilvert, Thomas**
  **24200 Sarlat la Canéda (FR)**
• **Millet, Jacques**
  **Sarlat la Canéda (FR)**
• **Bougherara, Chaabane**
  **2000 Frederiksberg C (DK)**
• **Chouarche, Olivier**
  **24200 Vitrac (FR)**

(56) References cited:
**WO-A1-02/089893**

## Description

## BACKGROUND

The present invention relates to a ureteral stent intended to maintain the flow of urine between the kidneys and the bladder of a patient.

In a person, urine secreted by the kidneys passes through the ureters to the bladder and is then evacuated from the body through the urethra during micturition. In a healthy person, the urine is evacuated from the kidney to the bladder in one direction, by means of peristaltic movements of the ureter.

Certain urological disorders or certain diseases may prevent this evacuation in the direction of the bladder. These disorders may in particular be due to the presence of a calculus or tumour or an obstruction of the pyeloureteral junction. In this case, the flow of urine to the bladder may be difficult or may no longer be possible at all. The urine remains in the kidney, which dilates and may cause nephritic colic. To remedy this disorder, a stent can be placed in the ureter in order to re-establish the function of the latter and to permit evacuation of the urine. A ureteral stent according to the preamble of claim 1 is known from the document WO 02/089893.

## SUMMARY

The ureteral stent of this application includes a body and a tail, the body having a renal area for placement in a kidney of a patient, a ureteral area for placement in at least part of a ureter of said patient, and a proximal area arranged at a proximal end of the body of the stent, the tail having at least one thread configured to end in the bladder of said patient.

The present invention relates to a stent which preserves the peristaltic movements of the ureter, thereby preventing reflux of urine in the direction of the kidneys. The stent avoids irritation of the bladder and irritation caused by friction in the ureter. The stent is therefore better tolerated by patients.

The ureteral stent as includes a tubular proximal area of cylindrical shape and annular cross section, and has a flexibility greater than the flexibility of the ureteral area of the stent.

The ureteral stent is useful for placement in a ureter of a patient suffering from a urological disorder or disease, such as a calculus, a tumour, or an obstruction of the pyeloureteral junction in particular. The stent extends as far as the kidney and has a curved renal area, the curved shape allowing the stent to be more effectively held in place in the kidney. The stent has a ureteral area inserted in the ureter of the patient. The ureteral area extends beyond the location of the urological disorder, and replaces the defective part of the ureter. One end of the stent is provided with a tail which comprises at least one thread configured to end in or near the bladder. The thread is suitable for permitting evacuation of the urine and has a sufficiently fine diameter to be virtually physically imperceptible to the patient.

## BRIEF DESCRIPTION OF THE DRAWINGS

The present invention and its advantages will be better understood with reference to the detailed description and with reference to the attached figures, in which:

- Figure 1 is a schematic view of a stent according to one embodiment when fitted in place in a patient;

- Figure 2 is an overall view of a ureteral stent according to one embodiment;

- Figure 3 is a sectional view of a part of the stent according to one embodiment;

- Figure 4 is a profile view of a stent according to an example;

- Figure 5 is an enlarged view of a detail of the proximal area of the example of Figure 4;

- Figure 6 is a top view of one example of the stent;

- Figure 7 is a sectional view of a part of the example of Figure 6;

- Figure 8 shows a stent according to one example of the invention;

- Figure 9 is a sectional view of a part of the proximal area of the example of Figure 8;

- Figure 10 is a perspective view of the part of the example of Figure 9;

- Figure 11 is a perspective view of a stent according to one example;

- Figure 12 shows a front view of the example of Figure 11;

- Figure 13 is a perspective view of a detail of the proximal area of the example of Figure 11;

- Figure 14 is a profile view of a stent according to one embodiment;

- Figure 15 is an enlarged view of part of the proximal area of the embodiment of Figure 14; and

- Figure 16 is an enlarged view of one embodiment of the proximal area of the stent.

**DETAILED DESCRIPTION**

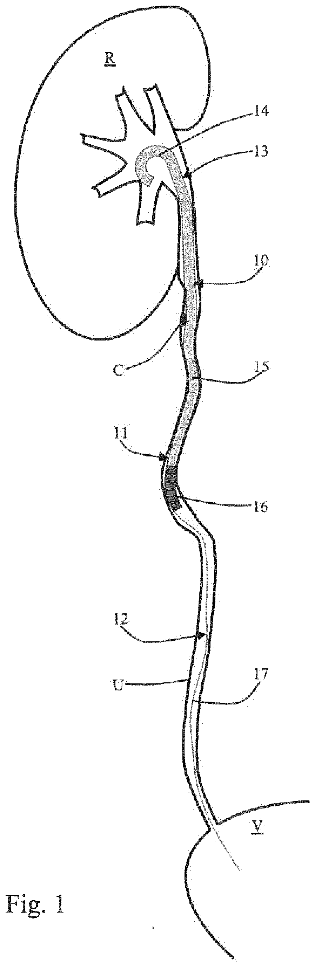

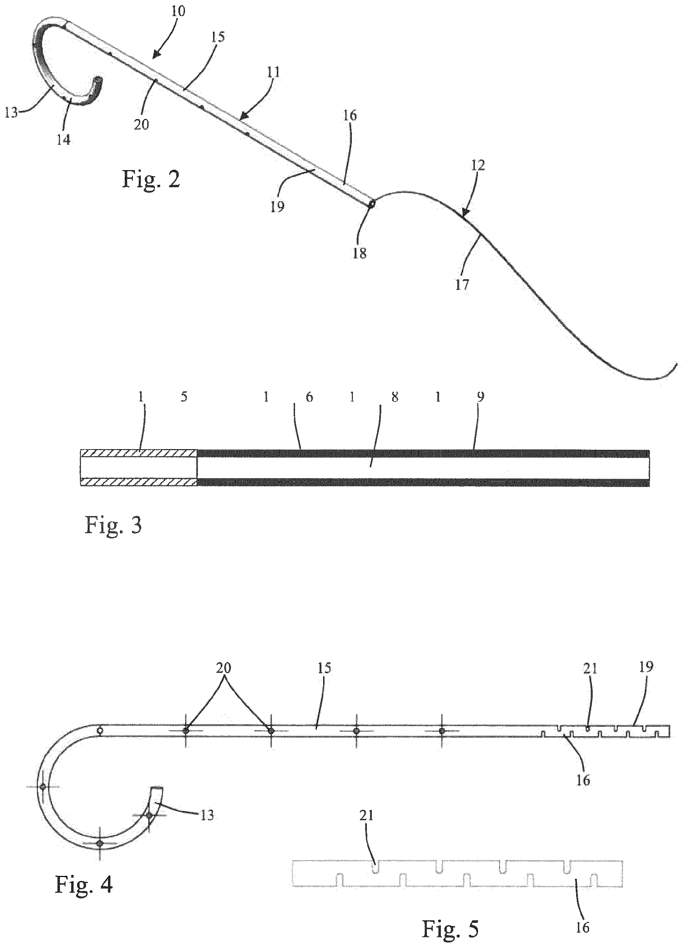

Figure 1 is a schematic view of one embodiment of a ureteral stent 10, placed in a patient presenting a calculus C. Figure 2 illustrates a similar stent before it is fitted in place in a patient.

With reference in particular to Figures 1 and 2, the ureteral stent 10 has a body 11 and a tail 12. The body 11 has a renal area 13 for placement in a kidney of a patient. The renal area 13 has a curved end 14 and improves retention of the stent. This part of the stent is physically imperceptible or virtually imperceptible to the patient and, as a result, does not cause any discomfort or pain.

The stent includes a ureteral area 15 for placement in a ureter U of the patient. Embodiments include stents manufactured with varying lengths of at least the ureteral area such as to accommodate variations in patient physiology. As has been indicated above, a ureteral stent of this kind is suitable for placement in a patient in cases of disease or obstruction in the area of the ureter. The obstruction may be due to the presence of a calculus C, as is illustrated by Figure 1, a tumour or a constriction in particular. The length of the ureteral area 15 of the stent should be sufficient to ensure that, after placement of the stent, the body 11 thereof extends beyond the site of the calculus C or the tumour in the direction of the bladder V.

The body 11 of the stent additionally has a proximal area 16 which is rigidly connected to the ureteral area 15, at the end away from the renal area 13. The proximal area 16 is situated in continuation of the ureteral area 15.

The tail 12 of the stent 10 is formed by at least one thread 17 or a suture configured to extend from the proximal area 16 in the direction of the bladder V, when the stent is placed in a patient.

In one embodiment, the thread permits flow of urine from the kidney R to the bladder V without permitting flow in the opposite direction from the bladder to the kidney. This avoids the aforementioned problems, particularly the sense of having to pass urine. The thread 17 causes a dilation of the ureter and, as a result, permits easier and therefore less painful evacuation of the calculi.

In one embodiment, the tail 12 includes a single thread, as illustrated in Figure 1. In one embodiment, it includes two or more threads. The tail 12 is configured to end in the bladder and extend a few centimetres into the bladder when the stent is placed in a patient. In one embodiment, the tail extends 5 to 6 centimetres into the bladder.

In embodiments wherein the tail is formed by two or more threads, the threads are free and independent of one another. In other embodiments, the threads are fixed to one another, for example by a knot. The knot is suitably located near the proximal area 16, in which case it will be positioned in the ureter during the use of the stent. Alternatively, the knot is located near the end of the threads away from the proximal area, in which case it will be positioned in the bladder during the use of the stent. Other ways of connecting the threads to one another are also acceptable, for example, but not limited to, braiding.

In one embodiment, the ureteral area 15 and the proximal area 16 are tubular and cylindrical, that is to say they have a substantially annular cross section, with a longitudinal channel 18 defining a lateral wall 19. In one example, the lateral wall has, at least in some areas, through-openings 20 which allow the urine to flow from outside the stent to the inside, and vice versa. In one embodiment, the longitudinal channel 18 opens out at the end of the proximal area 16, near the tail 12. In one embodiment, the renal area 13 also has a channel, and openings passing through the wall. The channel 18 serves, during the placement of the stent, for introduction of a guide and, during the use of the stent, for evacuation of urine.

In one embodiment, and with reference to Figures 1 and 3, the proximal area 16 of the stent is flexible. In particular, this proximal area is more flexible than the ureteral area 15.

In this application, flexibility is defined as the resistance to elastic deformation of a body. The more a body is flexible, the less the force that has to be applied to it to obtain a given deflection. In the present specification, stiffness or rigidity is considered the inverse of flexibility.

The flexibility can be measured in the following way: the specimen which flexibility is being determined is placed on two punctiform supports that are separated by a distance L. A downward force P is applied in the centre of the specimen. The deformation $U_y$ of the specimen is measured by measuring the movement of the centre of the specimen under the effect of the force P. This deformation, as a function of the force, allows a curve to be established. The coefficient of flexibility is defined as being the slope of the tangent to this curve, at the origin.

From the mathematical point of view, the coefficient of flexibility is defined by

$$K = \frac{L^3}{48} \cdot \frac{P}{|U_y|}$$

In one embodiment, the coefficient of flexibility of the proximal area of the stent is less than or equal to 200 N mm$^2$.

The more flexible the stent, the greater the flexibility and the lower the coefficient of flexibility.

Flexibility of the proximal area is suitably obtained by use of flexible materials and by providing the proximal area with a shape that gives it flexibility.

In one embodiment, the proximal area of the stent is configured to allow the body 11 of the stent to match the shape of a ureter U, in particular in the non-rectilinear parts of the ureter, during movements of the patient, especially movements caused by respiration. The stent and the ureter are configured to allow relative shifting between them . For this purpose, the proximal area 16 of the stent is sufficiently flexible to be able to follow the ureter.

In one embodiment, the ureteral area 15 of the stent has a flexibility allowing it to adapt to the sinuosities of the ureter. Materials suitable for producing the stent include polymers such as polyurethane, copolymers such as polyether block amide known by the name PEBA, polyamides, silicone, polyolefins sold under the names INFUSE™, VISTAMAXX™, QUEO™ or NOTIO™, polyamides, Poly Vinyl Chloride (PVC,) thermoplastic polyurethanes, aromatic polyethers, aromatic and aliphatic polyesters having a Shore hardness of generally between 25 and 95, compounds based on thermoplastic elastomers, vulcanized thermoplastic elastomers, mixtures and alloys based on thermoplastic polyurethane, polymers and copolymers sold under the names THERMOFLEX™, HYTRIL™, ARNITEL™, Ethylene Vinyl Acetate (EVA), and thermoplastic elastomers known by the acronyms SIS, SEBS, SEPS, SEEPS, SBS, SIBS or SIBSTAR.

In embodiments, an external diameter of the ureteral stents is between 1.5 mm and 4 mm.

In one embodiment, illustrated in Figures 1 and 3, the proximal area 16 is made of a flexible material. This material can be chosen from among different types of polymers such as polyurethane, copolymers such as polyether block amide (PEBA), polyvinyl chloride, polyamides or silicone in particular, or more generally from among the materials mentioned above for the ureteral area.

Suitable materials for the thread (or suture) include: polyethylene, polyamide, polyester, silk, steel, resorbable material (such as polyglactin acid), high-density polypropylene, meta-aramid and para-aramid, such as KEVLAR™ or NOMEX™.

In embodiments, the thread is configured to have a diameter ranging between 0.16 mm and 1.3 mm. In one embodiment, the diameter substantially equal to 0.2 mm.

When the stent 10 is implanted in a patient, the thread 17 serves to help the flow or urine from the kidney to the bladder, without permitting flow in the opposite direction. The thread also facilitates the evacuation of calculi, since it causes a dilation of the ureter. Another function of the thread is to permit the withdrawal of the stent when the stent is to be removed from the patient. In one embodiment, the thread 17 is sufficiently solid to allow the stent to be withdrawn by pulling on the thread. It is also possible to use two or more threads in order to for withdrawal of the stent.

In embodiments, the threads are fixed to the body 11 of the stent at different locations, particularly in the ureteral area 15 or in the proximal area 16 of the stent. One advantage of fixing the thread to the proximal area 16 is that the thread is always in contact with an edge of the proximal area 16. This permits an easy flow of urine. Moreover, this proximal area 16 is easily accessible in order to fix the thread to it.

Another advantage is the fact that a pull applied to the end of the proximal area eliminates the possibility of the latter folding or rolling up during the withdrawal of the stent.

In embodiments, the thread is fixed to the end of the ureteral area 15. This area is more rigid than the proximal area and it is therefore advantageously possible to apply a stronger pull to the thread.

In one embodiment, the thread can be fixed to the ureteral area 15 and to the proximal part 16. This makes it possible to fix the thread rigidly to the body of the stent, while at the same time providing for the thread to remain near the edge of the proximal area and avoid rolling up of the stent during withdrawal.

The fixing of the thread to the stent can be obtained in different ways. In one embodiment, the thread is passed through the wall of the body of the stent and knotted either to the wall or to another part of the thread. In one embodiment, the thread is adhesively bonded to the body of the stent. In one embodiment, the thread is fixed to the body by welding.

The flexibility of the material forming the proximal area 16 of the stent can be obtained in several different ways. In one embodiment, the proximal area 16 is made of a material different from that forming the rest of the body 11 of the stent 10. In one embodiment, the proximal area is made more flexible by subjecting it to a separate operation, for example a chemical action. In one embodiment, the proximal area of the stent is made of polyurethane, and the separate operation on the proximal area includes immersion in a plasticizer of the cyclohexanone type, the effect of which is to soften the material and increase the flexibility.

Alternatively, the stent can be produced with a body 11 made of a flexible material wherein the whole body of the stent except for the proximal area 16 is subjected to a hardening process. The hardening can be obtained by the action of a chemical component, by exposure to light, for example UV light, or by exposure to heating or cooling.

Figures 4 and 5 illustrate another example of a stent 10 having a flexible proximal area 16. In contrast to the embodiment illustrated in Figures 1 and 3, this flexibility is not provided by the material used or by a separate process, but instead by the shape of the proximal area 16. In one example, the proximal area has openings 21 passing through the lateral wall 19. In one example, the openings are transverse slits, that is to say slits arranged in a plane perpendicular to a longitudinal axis of the proximal area 16. The presence of these openings has the effect of allowing the proximal area to bend, which allows it to easily follow the sinuosities of the ureter, in particular when relative movements of the ureter and of the stent occur.

In one embodiment, the flexibility of the proximal area 16 is provided as a combination of its shape and by the choice of material. In one example, transverse openings 21 are formed in a proximal area 16 that is made of a flexible material.

In embodiments, the thread 17 or suture is configured to be fixed to the proximal area 16 or to the ureteral area 15. The advantages of this fixing are the same as those that have been described with reference to the embodiment in Figures 1 and 3.

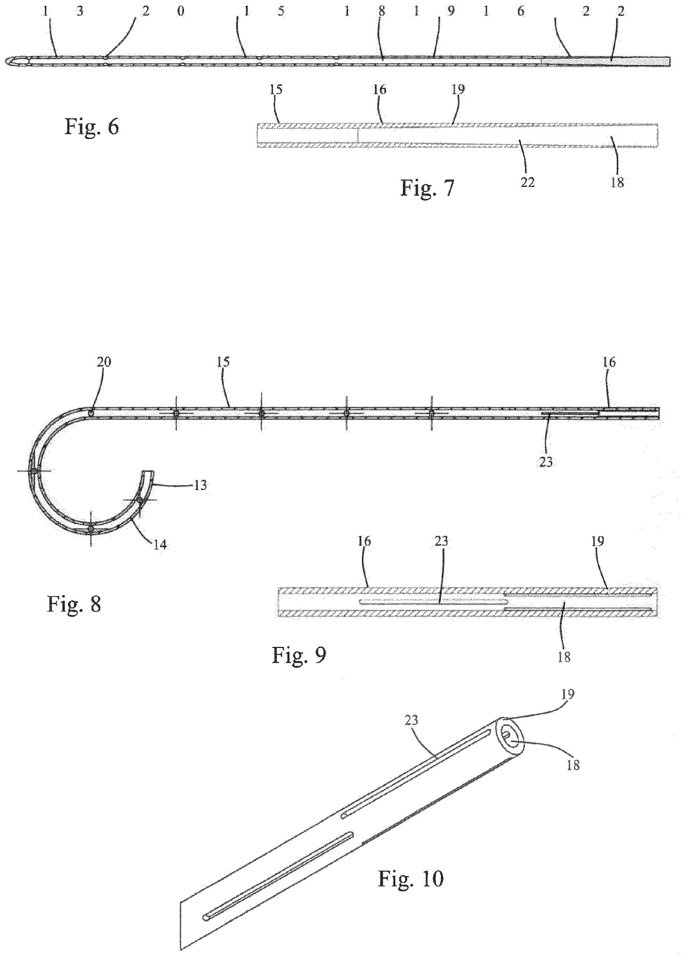

In the example illustrated in Figures 6 and 7, the proximal area 16 is cylindrical and includes a conical inner recess 22. This recess has an axis coincident with the longitudinal axis of the cylinder. The thickness of the lateral wall 19 forming the proximal area 16 decreases in the direction towards the tail of the stent. In this way, the end of the proximal area opposite the ureteral area has a very small thickness and, therefore, high flexibility. Thus, the proximal area can easily follow the movements of the ureter.

Figures 8, 9 and 10 show one example in which the proximal area 16 has through-openings made in the form of longitudinal slits 23 passing through the lateral wall 19. These longitudinal slits 23 allow the proximal area 16 to deform easily, so as to follow the shape of the ureter. In one example, the slits 23 do not extend all the way to the end of the proximal area 16, meaning that the end forms a complete ring.

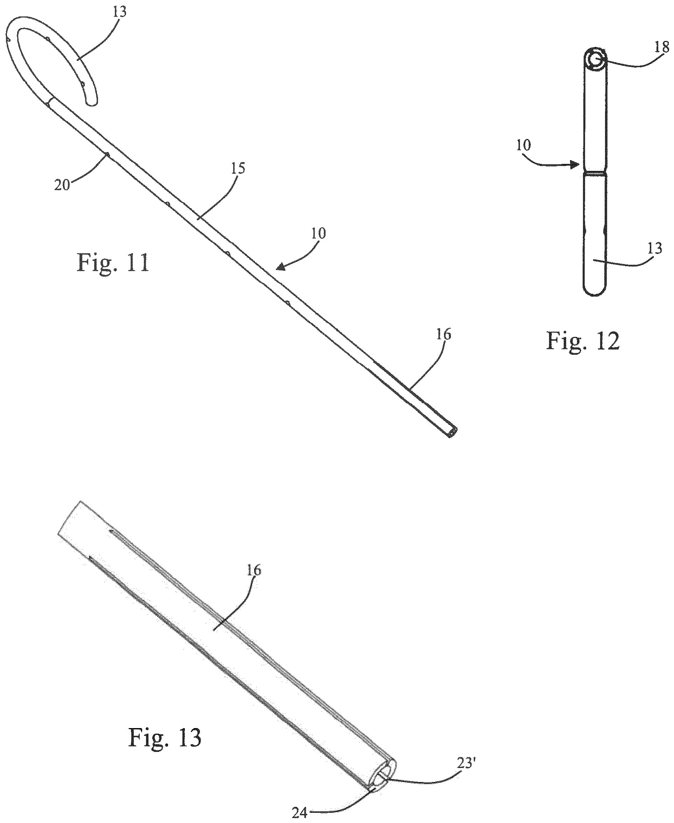

In one example illustrated in Figures 11, 12 and 13, the proximal area includes through-openings made in the form of longitudinal slits 23'. However, at least some of these slits extend all the way to the end of the stent in such a way as to form tongues 24 in the proximal area. The end therefore has a slit annular shape. The proximal area 16 is flexible and able to adapt to the shape of the ureter.

In one example, a thread is attached to the proximal area, more precisely to one of the tongues 24. In one embodiment, more than one thread is attached in such a way that all the tongues of this proximal area are connected to the threads forming the tail of the stent. This has the advantage of allowing the tongues to float in the ureter when the stent is in place in the patient in use, while also drawing these tongues together when the tail of the stent is pulled to extract it from the patient.

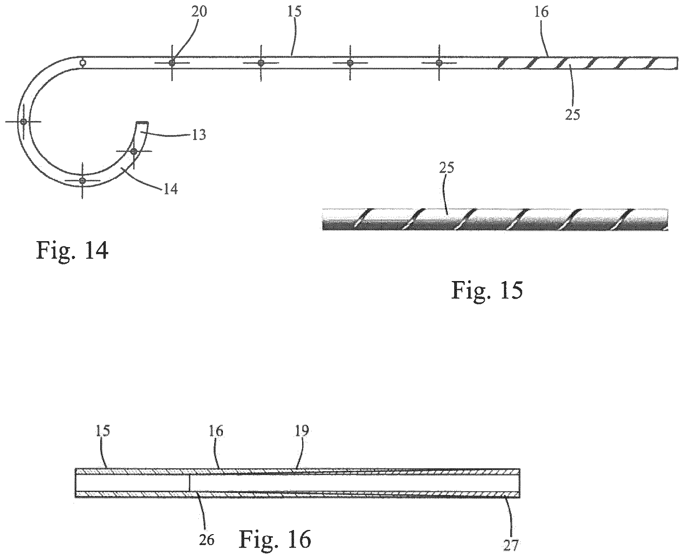

In Figures 14 and 15, the proximal area 16 of the stent is formed by a spiral 25. This spiral shape allows flexing of the proximal area and thus provides high flexibility. By virtue of the spiral shape, the stent can follow the sinuosities of the ureter of the patient. In one embodiment, the tail 12 of the stent is attached to one of the turns of the spiral of the proximal area 16. The turns of the spiral of the proximal area can be unwound to make withdrawal of the stent easier. In one embodiment including the spiral shape, the thread is fixed to the ureteral area 15 of the stent.

If unwinding of the spiral during withdrawal of the stent is not desired, in one embodiment the thread is fixed to several or all of the turns of the spiral 25 forming the proximal area. This makes it possible to retain the flexibility while at the same time avoiding too much deformation of the stent.

In one embodiment, two threads are provided, one thread being fixed to the proximal area 16 and the other to the ureteral area 15. The thread fixed to the proximal area is able to guide the urine during the use of the stent and to guide the proximal part during the withdrawal of the stent. The thread fixed to the ureteral area makes it possible to retract the body of the stent without deforming it and also makes it possible to limit the deformation of the proximal area during withdrawal.

In one embodiment illustrated in Figure 16, the proximal area 16 is produced, for example, by co-extrusion of two materials having different degrees of flexibility. The thickness of the first material 26, having a defined coefficient of flexibility and being used to form the ureteral area 15, decreases in the direction of the tail end of the stent, while the thickness of the second material 27, having a coefficient of flexibility lower than that of the first material, increases in the direction of the tail end of the stent, in such a way that the total thickness of the two materials remains constant. In other words, less flexible material is progressively replaced by more flexible material in the direction of the free end of the stent.

A ureteral stent has been described which permits evacuation of urine from a patient while at the same time preventing reflux of the urine in the direction of flow towards the kidneys. The stent is configured to be placed to permit easier evacuation of calculi. The stent is virtually physically imperceptible to the patient due to the flexibility of the proximal area. The thread too is fine and flexible, such that it is practically unnoticed by the patient.

**Claims**

1. A ureteral stent (10) comprising:

   a body (11) having a renal area (13) configured to be placed in a kidney of a patient, a ureteral area (15) connected to the renal area (13) and configured to be placed in a ureter of the patient, and a proximal area (16) connected to the ureteral area (15) and located at a proximal end of the body (11); and
   a tail (12) comprising a thread (17) connected to the proximal end of the body (11) of the stent (10); wherein the proximal area (16) is tubular and is provided with a first flexibility that is greater than a second flexibility of the ureteral area (15) of the stent, **characterized in that**
   the tubular proximal area (16) is configured as a spiral shape (25).

2. The ureteral stent according to claim 1, wherein the proximal area (16) of the stent (10) comprises a material that is more flexible than a material forming the ureteral area (15) of the stent (10).

3. The ureteral stent according to claim 2, wherein the proximal area (16) of the stent (10) is fabricated from two materials each having a different flexibility and the proximal area (16) has a longitudinal channel (18) of constant diameter defining a lateral wall (19) of constant thickness.

4. The ureteral stent according to claim 3, wherein a first of the materials is less flexible than a second of the two materials, and a thickness of the first material decreases towards the tail (12) of the stent (10) and a thickness of the second material increases towards the tail (12) of the stent (10).

5. The ureteral stent according to claim 1, wherein the tail (12) is rigidly connected to the proximal area (16).

6. The ureteral stent according to claim 1, wherein the tail (12) is rigidly connected to the ureteral area (15).

7. The ureteral stent according to claim 1, wherein a body portion of the stent in the proximal area (16) has a coefficient of flexibility equal to or below 200 N mm$^2$.

**Patentansprüche**

1. Harnleiterstent (10), umfassend:

   einen Körper (11) mit einem Nierenbereich (13), der ausgestaltet ist, um in einer Niere eines Patienten platziert zu werden, einem Harnleiterbereich (15), der mit dem Nierenbereich (13) verbunden ist und ausgestaltet ist, um in einem Harnleiter des Patienten platziert zu werden, und einem proximalen Bereich (16), der mit dem Harnleiterbereich (15) verbunden ist und sich an einem proximalen Ende des Körpers (11) befindet; und
   einen Schwanz (12), der ein Gewinde (17) umfasst, das mit dem proximalen Ende des Körpers (11) des Stents (10) verbunden ist; wobei der proximale Bereich (16) rohrförmig ist und mit einer ersten Flexibilität ausgestattet ist, die größer als eine zweite Flexibilität des Harnleiterbereichs (15) des Stents ist, **dadurch gekennzeichnet, dass** der rohrförmige proximale Bereich (16) als Spiralform (25) ausgestaltet ist.

2. Harnleiterstent nach Anspruch 1, wobei der proximale Bereich (16) des Stents (10) ein Material umfasst, das flexibler als ein Material ist, welches den Harnleiterbereich (15) des Stents (10) bildet.

3. Harnleiterstent nach Anspruch 2, wobei der proximale Bereich (16) des Stents (10) aus zwei Materialien gefertigt ist, die jeweils eine unterschiedliche Flexibilität aufweisen, und wobei der proximale Bereich (16) einen längsgerichteten Kanal (18) mit konstantem Durchmesser aufweist, der eine Seitenwand (19) mit konstanter Dicke definiert.

4. Harnleiterstent nach Anspruch 3, wobei ein erstes der Materialien weniger flexibel als ein zweites der beiden Materialien ist, und eine Dicke des ersten Materials in Richtung des Schwanzes (12) des Stents (10) abnimmt, und eine Dicke des zweiten Materials in Richtung des Schwanzes (12) des Stents (10) zunimmt.

5. Harnleiterstent nach Anspruch 1, wobei der Schwanz (12) starr mit dem proximalen Bereich (16) verbunden ist.

6. Harnleiterstent nach Anspruch 1, wobei der Schwanz (12) starr mit dem Harnleiterbereich (15) verbunden ist.

7. Harnleiterstent nach Anspruch 1, wobei ein Körperabschnitt des Stents in dem proximalen Bereich (16) einen Flexibilitätskoeffizienten gleich oder kleiner als 200 N mm$^2$ aufweist.

**Revendications**

1. Endoprothèse urétérale (10) comprenant :

   un corps (11) possédant une zone rénale (13) conçue pour être placée dans un rein d'un patient, une zone urétérale (15) reliée à la zone rénale (13) et conçue pour être placée dans un uretère du patient, et une zone proximale (16) reliée à la zone urétérale (15) et située au niveau d'une extrémité proximale du corps (11) ; et
   une queue (12) comprenant un fil (17) relié à l'extrémité proximale du corps (11) de l'endoprothèse (10) ; la zone proximale (16) étant tubulaire et étant dotée d'une première souplesse qui est supérieure à une seconde souplesse de la zone urétérale (15) de l'endoprothèse, **caractérisée en ce que**
   la zone proximale tubulaire (16) est conçue en forme de spirale (25).

2. Endoprothèse urétérale selon la revendication 1, dans laquelle la zone proximale (16) de l'endoprothèse (10) comprend un matériau qui est plus souple qu'un matériau formant la zone urétérale (15) de l'endoprothèse (10).

3. Endoprothèse urétérale selon la revendication 2, dans laquelle la zone proximale (16) de l'endoprothèse (10) est fabriquée à partir de deux matériaux ayant chacun une souplesse différente et la zone proximale (16) possède un canal longitudinal (18) de diamètre constant définissant une paroi latérale (19) d'épaisseur constante.

4. Endoprothèse urétérale selon la revendication 3, dans laquelle un premier des matériaux est moins souple qu'un second des deux matériaux, et une épaisseur du premier matériau diminue vers la queue (12) de l'endoprothèse (10) et une épaisseur du second matériau augmente vers la queue (12) de l'endoprothèse (10).

5. Endoprothèse urétérale selon la revendication 1, dans laquelle la queue (12) et la zone proximale (16) sont solidarisées.

6. Endoprothèse urétérale selon la revendication 1, dans laquelle la queue (12) et la zone urétérale (15) sont solidarisées.

7. Endoprothèse urétérale selon la revendication 1, dans laquelle une partie corps de l'endoprothèse dans la zone proximale (16) présente un coefficient de souplesse inférieur ou égal à 200 N mm$^2$.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

20    15                16

13

14

**Fig. 14**

25

25

**Fig. 15**

15        16        19

26    **Fig. 16**        27

**EP 2 977 074 B1**

**Patent documents cited in the description**

- WO 02089893 A